# EUROPEAN PATENT APPLICATION

(11) **EP 4 579 239 A1**
(43) Date of publication of application: **02.07.2025**
(21) Application number: 24220833.8
(22) Date of filing: 18.12.2024
(51) Int. Cl.: G01N 33/58, G01N 33/76, C09K 11/07

(54) **CHEMILUMINESCENT SUBSTRATE SOLUTION AND CHEMILUMINESCENCE DETECTION METHOD**

(30) Priority: 29.12.2023 CN 202311870910
(71) Applicant: Shenzhen Mindray Bio-Medical Electronics Co., Ltd., Shenzhen, Guangdong 518057 (CN)
(72) Inventor: WANG, Di, Shenzhen, 518057 (CN); LIAO, Zhiwei, Shenzhen, 518057 (CN); SONG, Shanshan, Shenzhen, 518057 (CN); LI, Ke, Shenzhen, 518057 (CN)
(74) Representative: KIPA AB

(57) **Abstract**

The present disclosure relates to a chemiluminescent substrate solution and a chemiluminescence detection method. The chemiluminescent substrate solution includes a chemiluminescent substrate, a fluorescein, and a water-soluble polymeric quaternary ammonium salt, wherein the chemiluminescent substrate is selected from chlorinated dioxetane compounds with a spiroadamantane substituent. The chemiluminescent substrate solution has a wider linear detection range.

## Description

### TECHNICAL FIELD

The present disclosure relates to the field of immunoassays for in-vitro diagnosis, and in particular to an alkaline-phosphatase-catalyzed chemiluminescent substrate solution and a chemiluminescence detection method.

### BACKGROUND

Chemiluminescence refers to a phenomenon that in the process of a chemical reaction, a specific substance absorbs a portion of chemical energy and reaches an excited state, and the energy released in the process of returning to a ground state is released in the form of photons, resulting in luminescence. Chemiluminescence immunoassay (CLIA) refers to an analytic technology that combines a high-sensitivity chemiluminescence technology with high-specificity immune response for the detection of antigens, antibodies, hormones, fatty acids, vitamins, drugs and the like. Chemiluminescence immunoassay has both the high sensitivity of chemiluminescence and the characteristics of simple operation and rapid response of enzyme-linked immune response. It is easy to achieve standardized experimental operations, and now has been widely used in biological and medical studies and clinical diagnosis.

In current immunoassays, some analytes have significant concentration differences in clinical samples, which may even differ by orders of magnitude of up to 10⁵-10⁶. Currently, the method of diluting before testing is used for high-concentration samples, which has disadvantages such as a long test cycle, high testing costs, significant interference due to dilution, and cumbersome operations.

Therefore, there is a need for further improvements in the detection method for such clinically tested items.

### SUMMARY

An object of the present disclosure is to provide a chemiluminescent substrate solution with wide linearity, and a chemiluminescence detection method.

Therefore, a first aspect of the present disclosure provides a chemiluminescent substrate solution including a chemiluminescent substrate, a fluorescein, and a water-soluble polymeric quaternary ammonium salt, wherein the chemiluminescent substrate is selected from chlorinated dioxetane compounds with a spiroadamantane substituent.

According to some embodiments, the chlorinated dioxetane compounds with a spiroadamantane substituent are ADP-STAR and CDP-STAR.

According to some embodiments, the water-soluble polymeric quaternary ammonium salt is selected from polyvinyl-containing quaternary ammonium salts. Preferably, the water-soluble polymeric quaternary ammonium salt is selected from at least one of polyvinylbenzyl-trimethylammonium chloride, polyvinylbenzyl-benzyldimethylammonium chloride, and polyvinylbenzyl-tributylammonium chloride.

According to some embodiments, the ratio of the number of photons generated per second at the upper limit of linear detection of the chemiluminescent substrate solution to that at the lower limit of linear detection thereof is 30,000 or more, preferably 50,000 or more.

According to some embodiments, the lower limit of linear detection of the chemiluminescent substrate solution is less than or equal to 3,000 photons/second, and the upper limit of linear detection thereof is larger than or equal to 100M photons/second.

According to some preferred embodiments, the lower limit of linear detection of the chemiluminescent substrate solution is 1,000-3,000 photons/second, preferably 1,000-2,000 photons/second; and the upper limit of linear detection of the chemiluminescent substrate solution is 100M-200M photons/second, preferably 120M-200M photons/second.

According to some embodiments, the chemiluminescent substrate solution includes 50-500 mg/L of the chemiluminescent substrate, 30-500 mg/L of the fluorescein, and 1-10 g/L of the water-soluble polymeric quaternary ammonium salt.

According to some embodiments, the chemiluminescent substrate solution includes 150-250 mg/L of the chemiluminescent substrate, 150-250 mg/L of the fluorescein, and 3-7 g/L of the water-soluble polymeric quaternary ammonium salt.

According to some embodiments, the chemiluminescent substrate solution further includes a buffer and a preservative.

According to some embodiments, the buffer is selected from at least one of Tris-HCl, AMP-HCl, AMPD-HCl, DEA-HCl, CHES-HCl, boric acid-NaOH, glycine-NaOH buffer systems, and preferably the buffer is selected from an AMP-HCl buffer system.

According to some embodiments, the preservative is selected from at least one of sodium azide, Proclin series, potassium sorbate, and sodium benzoate.

According to some embodiments, the chemiluminescent substrate solution is chemiluminescent through the catalysis of an alkaline phosphatase.

According to some embodiments, the concentration of the alkaline phosphatase is as low as 10⁻¹⁹ mol/L.

According to some embodiments, the chemiluminescent substrate solution is used to detect human chorionic gonadotropin (HCG) in a sample to be tested.

According to some embodiments, the concentration of the human chorionic gonadotropin (HCG) in the sample to be tested ranges from 1 mIU/mL to 200,000 mIU/mL. Preferably, the sample to be tested is a blood sample.

According to some embodiments, the chemiluminescent substrate solution is used to detect the human chorionic gonadotropin (HCG) in a sample to be tested using a double-antibody sandwich method.

According to some embodiments, in the double-antibody sandwich method, superparamagnetic particles coated with an HCG antibody are used as a capture reagent, and an alkaline-phosphatase-labeled HCG antibody is used as a marker.

According to a second aspect of the present disclosure, a chemiluminescence detection method is provided, which includes steps of:
mixing a sample to be tested with a detection reagent including a capture reagent and an alkaline phosphatase marker that are capable of binding to an analyte to obtain an alkaline-phosphatase-labeled immune complex;
mixing the alkaline-phosphatase-labeled immune complex with the chemiluminescent substrate solution of any embodiment in the first aspect above to obtain a mixed solution; and
determining the light signals of the mixed solution, and obtaining the analysis result of the analyte in the sample to be tested on the basis of the light signals.

According to a third aspect of the present disclosure, a chemiluminescence detection method is provided, which includes steps of:
mixing a sample to be tested with a detection reagent including a capture reagent and an alkaline phosphatase marker that are capable of binding to an analyte to obtain an alkaline-phosphatase-labeled immune complex;
mixing the alkaline-phosphatase-labeled immune complex with a chemiluminescent substrate solution to obtain a mixed solution; and
determining the light signals of the mixed solution, and obtaining the analysis result of the sample to be tested on the basis of the light signals.

The chemiluminescent substrate solution includes a chemiluminescent substrate, a fluorescer, and a surfactant.

The ratio of the number of photons generated per second at the upper limit of linear detection of the chemiluminescent substrate solution to that at the lower limit of linear detection thereof is 30,000 or more; alternatively, the lower limit of linear detection of the chemiluminescent substrate solution is less than or equal to 2,000 photons/second, and the upper limit of linear detection thereof is larger than or equal to 100M photons/second.

According to some embodiments, the ratio of the number of photons generated per second at the upper limit of linear detection of the chemiluminescent substrate solution to that at the lower limit of linear detection thereof is 50,000 or more; alternatively, the lower limit of linear detection of the chemiluminescent substrate solution is 1,000-3,000 photons/second, preferably 1,000-2,000 photons/second, and the upper limit of linear detection of the chemiluminescent substrate solution is 100M-200M photons/second, preferably 120M-200M photons/second.

According to some embodiments, determining the light signals of the mixed solution includes determining the light signals of the mixed solution using a photometer, with the lower limit of linear detection of the photometer being less than or equal to 2,000 photons/second, and the upper limit of linear detection of the photometer being larger than or equal to 100M photons/second.

According to some embodiments, the chemiluminescent substrate is a dioxetane compound. Preferably, the dioxetane compound is selected from chlorinated dioxetane compounds with a spiroadamantane substituent; the fluorescer is selected from a fluorescein and/or a carboxyl-substituted fluorescein; and the surfactant is selected from water-soluble polymeric quaternary ammonium salt surfactants.

According to some preferred embodiments, the chlorinated dioxetane compounds with a spiroadamantane substituent are ADP-STAR and CDP-STAR; and the carboxyl-substituted fluorescein is 5-carboxyfluorescein, 6-carboxyfluorescein, and 5(6)-carboxyfluorescein.

According to some preferred embodiments, the water-soluble polymeric quaternary ammonium salt is a polyvinyl-containing quaternary ammonium salt, preferably at least one of polyvinylbenzyl-trimethylammonium chloride, polyvinylbenzyl-benzyldimethylammonium chloride, and polyvinylbenzyl-tributylammonium chloride.

According to some embodiments, the chemiluminescent substrate solution includes 50-500 mg/L of the chemiluminescent substrate, 30-500 mg/L of the fluorescer, and 1-10 g/L of the water-soluble polymeric quaternary ammonium salt. Preferably, the chemiluminescent substrate solution includes 150-250 mg/L of the chemiluminescent substrate, 150-250 mg/L of the fluorescer, and 3-7 g/L of the water-soluble polymeric quaternary ammonium salt.

According to some embodiments, in the above method, the analyte is human chorionic gonadotropin (HCG). Preferably, the concentration of the HCG in the sample to be tested ranges from 1 mIU/mL to 200,000 mIU/mL. More preferably, the method is a double-antibody sandwich method. Especially preferably, the capture reagent is superparamagnetic particles coated with an HCG antibody.

The chemiluminescent substrate solution of the present disclosure, which combines a chemiluminescent substrate, a fluorescer, and a surfactant, can achieve a ratio of the number of photons generated per second at the upper limit of detection to that at the lower limit of detection reaching 50,000 or more, which may even reach a wide linearity of 60,000 or more in a preferred embodiment. In this way, both a low-concentration sample and a high-concentration sample can be precisely detected by using an enzyme (such as alkaline-phosphatase) catalyzed luminescence immunoassay system, without the need for identifying whether the concentration of the analyte in the sample exceeds the detection limit and then diluting, thus simplifying the detection procedures and improving the detection efficiency.

### BRIEF DESCRIPTION OF THE DRAWINGS

FIGS. 1A to 1D show the linear fitted curves of the luminescence values of the chemiluminescent substrate solutions 1-1 to 1-4 of example 1 in an alkaline phosphatase system.
FIGS. 2A to 2D show the linear fitted curves of the luminescence values of the chemiluminescent substrate solutions 2-1 to 2-4 of example 2 in an alkaline phosphatase system.
FIGS. 3A to 3D show the linear fitted curves of the luminescence values of the chemiluminescent substrate solutions 3-1 to 3-4 of example 3 in an alkaline phosphatase system.
FIGS. 4A to 4D show the linear fitted curves of the luminescence values of the chemiluminescent substrate solutions 4-1 to 4-4 of example 4 in an alkaline phosphatase system.
FIGS. 5A to 5D show the linear fitted curves of the luminescence values of the chemiluminescent substrate solutions 5-1 to 5-4 of example 5 in an alkaline phosphatase system.

### DESCRIPTION OF THE PREFERRED EMBODIMENTS

The technical solutions of the disclosure will be clearly and completely described below in conjunction with specific embodiments and drawings. Obviously, the embodiments described are merely some of the embodiments of the disclosure rather than all the embodiments. Based on the embodiments in the disclosure, all the other embodiments that would have been obtained by those of ordinary skill in the art without any inventive effort shall fall within the scope of protection of the disclosure.

Throughout the specification, unless otherwise specified, the terms used herein should be understood as the meanings commonly used in the art. Therefore, unless otherwise defined, all the technical and scientific terms used herein have the same meaning as commonly understood by those of skill in the art to which the disclosure belongs. In the event of a conflict, this specification takes precedence.

In this application, the terms "comprise", "include" or any other variation thereof are intended to cover non-exclusive inclusion, so that a method or product comprising a series of elements comprises not only explicitly recorded elements, but also other elements not explicitly listed, or elements inherent in implementing the method or product.

Unless otherwise specified, the singular forms "a/an" and "the/said" as used herein include the plural of the noun referred to.

Chemiluminescence Immunoassay (CLIA) is an immunoassay technology that combines a chemiluminescent system with the immune response to detect trace antigens or antibodies with high sensitivity. However, it is always challenging to achieve both sensitivity and a wide linear range in measurement.

As described above, in current immunoassays, the concentrations of some analytes such as human chorionic gonadotropin (HCG) in clinical samples differ greatly, and may even differ by orders of magnitude of up to 10⁵-10⁶. The content of HCG may vary from less than 3 mIU/ml to about 2,000,000 mIU/ml in non-pregnant and pregnant individuals. A mainstream kit for quantitative detection of HCG using a chemiluminescence method has a detection range with a lower limit of 0.1-2 mIU/ml and an upper limit of 1,000-15,000 mIU/ml. After an investigation of the concentration distribution of the HCG samples from several hospitals over a year, it was found that in clinical HCG test samples, the proportion of the samples with a concentration greater than 15,000 mIU/ml was as high as 40%. When considering the precise detection of low-concentration samples, if the sample is tested as received, approximately 40% of the samples will exceed the upper limit of detection of the existing method. When such a sample is found, it needs to be diluted and then retested. This seriously leads to significant time, labor, and reagent consumption, low detection efficiency, and high detection costs.

Therefore, in the clinical detection of items such as HCG, it is necessary to provide a detection reagent and a detection method with wide linearity to make the detection simpler, more efficient, and more precise.

In order to enable a wide linear immunoassay method for samples with a broad concentration range of the analyte, the present disclosure provides a chemiluminescent substrate solution with wide linear luminescence values and a chemiluminescence immunoassay method.

According to a first aspect of the present disclosure, provided is a chemiluminescent substrate solution, which is characterized in that the chemiluminescent substrate solution includes a chemiluminescent substrate, a fluorescein, and a water-soluble polymeric quaternary ammonium salt, wherein the chemiluminescent substrate is selected from chlorinated dioxetane compounds with a spiroadamantane substituent.

A chemiluminescent substrate refers to a compound that participates in energy transfer in a chemiluminescent reaction and finally releases energy in the form of emitting photons, which is also known as a chemiluminescent reagent or a luminescent substrate. Dioxetane compounds are enzymatic glow-type chemiluminescent substrates and are ultra-sensitive substrates for an alkaline phosphatase (AP). In a suitable buffer solution, with the catalytic hydrolysis effect of the alkaline phosphatase, the dioxetane compound will decompose and emit signals that may last for 20 hours or longer, which is an ideal chemiluminescent substance.

The present inventors found that a chemiluminescent substrate solution with the ratio of the number of photons generated per second at the upper limit of detection to that at the lower limit of detection reaching 30,000 or more, even 50,000 or more, can be obtained from a chlorinated dioxetane compound with a spiroadamantane substituent when combined with a specific chemiluminescence enhancer such as a fluorescein or a carboxylic acid derivative thereof in the presence of a cationic surfactant of water-soluble polymeric quaternary ammonium salt, which can be conveniently applied to the immunoassay of samples such as HCG in which the concentrations of the analyte differ by orders of magnitude of up to 10⁵-10⁶.

The "dioxetane compounds with a spiroadamantane substituent" mentioned herein refer to compounds based on the structure below:

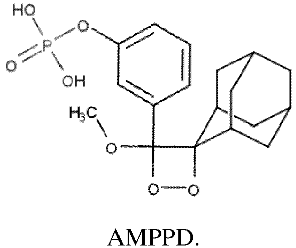

The examples include, but are not limited to, the following compounds:
AMPPD - (3-(2'-spiroadamatane)-4-methoxy-4-(3"-phosphoryloxy)-phenyl-1,2-dioxetane, CAS = 122341-56-4),
CSPD - (3-(2'-(spiro-5-chloroadamantane))-4-methoxy-4-(3"-phosphoryloxy)-phenyl-1,2-dioxetane, CAS = 142456-88-0),
ADP-STAR - (3-(2'-spiroadamatane)-4-methoxy-4-(3"-phosphoryloxy-4"-chloro)- phenyl-1,2-dioxetane, CAS = 189942-84-5),
CDP-STA - (3-(2'-(spiro-5-chloroadamantane))-4-methoxy-4-(3"-phosphoryloxy-4"-chloro)-phenyl-1,2-dioxetane, CAS = 160081-62-9), and
TFE-AMPPD - (3-(2'-spiroadamatane)-4-trifluoroethoxy-4-(3"-phosphoryloxy)-phenyl-1,2-dioxetane).

The "chlorinated dioxetane compounds with a spiroadamantane substituent" refer to those including at least one chlorine atom in the structure of AMPPD.

In some embodiments, the chlorinated dioxetane compound with a spiroadamantane substituent is one of ADP-STAR and CDP-STAR.

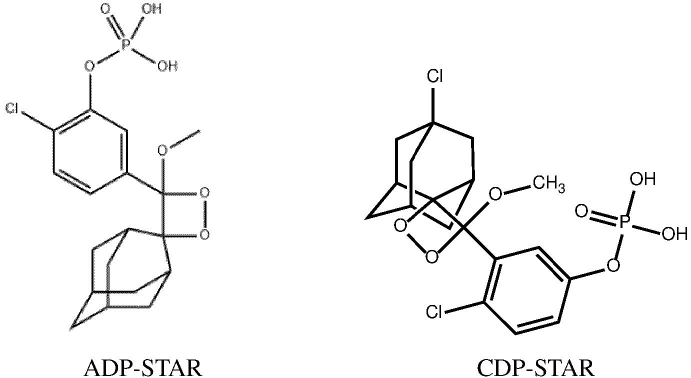

In a specific embodiment, the chemiluminescent substrate in the chemiluminescent substrate solution may be present in the form of a salt of the aforementioned compound. According to some embodiments, the salt may be an alkali metal salt, such as a sodium salt.

These dioxetane compounds with a chloro group, particularly ADP-STAR and CDP-STAR, when combined with a fluorescein or a carboxyl derivative thereof and a water-soluble polymeric quaternary ammonium salt in an alkaline-phosphatase-catalyzed system can unexpectedly provide sufficiently low sensitivity while maintaining linearity within a wide range of luminescence values.

The fluorescer and the surfactant can improve the chemiluminescence efficiency. Here, the surfactant forms micelles in the solution to protect the chemiluminescent substrate, resulting in the reduction of quenching reaction of the chemiluminescent substrate in an aqueous solution. As a photon acceptor, the fluorescer receives the energy of photons in a system through the effect of energy transfer and is excited to generate light signals, thus enhancing the luminescence efficiency.

The fluorescer in the chemiluminescent substrate solution of the present disclosure is at least one of a fluorescein and a carboxyl-substituted fluorescein.

The carboxyl-substituted fluorescein includes a compound represented by general formula I:

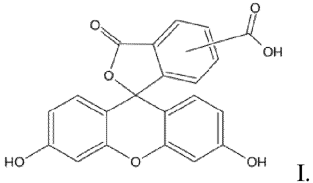

Specifically, the carboxyfluorescein represented by general formula I may be 5-carboxyfluorescein, 6-carboxyfluorescein, or 5(6)-carboxyfluorescein. Here,
the 5-carboxyfluorescein is 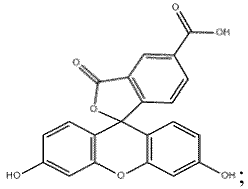
the 6-carboxyfluorescein is 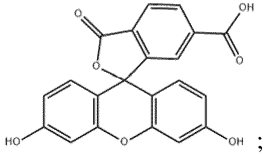
the 5(6)-carboxyfluorescein is a mixture of the 5-carboxyfluorescein and the 6-carboxyfluorescein in any ratio.

In the chemiluminescent substrate solution, the fluorescer is a fluorescein. In other embodiments, the fluorescer may be any carboxyl-substituted fluorescein, particularly one of 5-carboxyfluorescein, 6-carboxyfluorescein, and 5(6)-carboxyfluorescein.

The fluorescein and a carboxyl derivative thereof can improve the chemiluminescence efficiency of the chemiluminescent system. The aforementioned carboxyl derivative can, in addition to effectively improving the chemiluminescence efficiency of the chemiluminescent system, reduce the time required to reach a plateau and improve the sensitivity.

In the chemiluminescent substrate solution, the surfactant is a cationic surfactant of water-soluble polymeric quaternary ammonium salt. According to an embodiment, the water-soluble polymeric quaternary ammonium salt is selected from polyvinyl-containing quaternary ammonium salts. Such quaternary ammonium salts are, for example, polyvinylbenzyl-trihydrocarbylammonium halides. Here, the three hydrocarbyl groups substituted on the N atom, which may be the same or different, for example, may be independently selected from methyl, ethyl, propyl, butyl, pentyl, benzyl, and the like. In a specific embodiment, the water-soluble polymeric quaternary ammonium salt is selected from at least one of polyvinylbenzyl-trimethylammonium chloride, polyvinylbenzyl-benzyldimethylammonium chloride, and polyvinylbenzyl-tributylammonium chloride.

The cationic surfactant of water-soluble polymeric quaternary ammonium salt may likewise form micelles in an aqueous solution, with the chemiluminescent substrate located at the hydrophobic portion inside the micelles, and the fluorescer located at the hydrophilic portion outside the micelles, which is more conducive to the energy transfer from the chemiluminescent substrate to the fluorescer to enhance the light signals. The polymeric quaternary ammonium salt has a longer alkane chain compared to a small-molecule quaternary ammonium salt, which can prevent the quenching of the chemiluminescent substrate in an aqueous solution more effectively. Additionally, the polymeric quaternary ammonium salt may also decrease a background signal and increase a detection signal. By comparison, the small-molecule quaternary ammonium salt under the same conditions has a high background signal value and a lower detection signal, failing to achieve a wide linear detection range.

In a specific embodiment, the chemiluminescent substrate solution includes ADP-STAR or CDP-STAR as a chemiluminescent substrate, a fluorescein, and a water-soluble polymeric quaternary ammonium salt.

In another specific embodiment, the chemiluminescent substrate solution includes ADP-STAR, a fluorescein or a carboxyl-substituted fluorescein as a fluorescer, and a water-soluble polymeric quaternary ammonium salt.

The above specific combination of the chemiluminescent substrate, the fluorescer, and the surfactant provides a chemiluminescent substrate solution with wide linear luminescence values. The chemiluminescent substrate solution in an alkaline phosphatase system may achieve a linear range with a lower limit of 3,000 photons/second or less and an upper limit of 100,000,000 (100M) photons/second or more. As an example, the chemiluminescent substrate solution in an alkaline phosphatase system may achieve a linear range with a lower limit of 1,000-3,000 photons/second and an upper limit of 100,000,000 (100M) - 200,000,000 (200M) photons/second. As shown in the following examples, the chemiluminescent substrate solutions in an alkaline phosphatase system may achieve a linear range with a background luminescence value of 1,000-2,000 photons/second and an upper limit of 120,000,000 (120M) - 200,000,000 (200M) photons/second. It is understood that the ranges of the lower limit and the upper limit mentioned above are merely exemplary, and a lower limit of fewer photons/second or an upper limit of more photons/second may be obtained by, for example, adjusting the detection apparatus or signal processing method.

In some embodiments, the ratio of the number of photons generated per second at the upper limit of detection to that at the lower limit of detection of the chemiluminescent substrate solution is 30,000 or more, for example, 50,000 or more, even 60,000 or more. In some embodiments, the ratio of the number of photons generated per second at the upper limit of detection to that at the lower limit of detection of the chemiluminescent substrate solution is 1,000,000 or less, such as 800,000 or less, 600,000 or less, 500,000 or less, even 400,000 or less. As an example, the ratio of the number of photons generated per second at the upper limit of detection to that at the lower limit of detection of the chemiluminescent substrate solution is 30,000-1,000,000, 30,000-800,000, 30,000-500,000, 50,000-1,000,000, 50,000-800,000, 50,000-500,000, etc.

In some embodiments, the chemiluminescent substrate solution includes 50-500 mg/L of the chemiluminescent substrate. Preferably, the chemiluminescent substrate solution includes 150-250 mg/L, and more preferably 200 mg/L of the chemiluminescent substrate. For example, the chemiluminescent substrate solution includes 150 mg/L, 160 mg/L, 170 mg/L, 180 mg/L, 190 mg/L, 200 mg/L, 210 mg/L, 220 mg/L, 230 mg/L, 240 mg/L, and 250 mg/L of the chemiluminescent substrate.

In some embodiments, the chemiluminescent substrate solution includes 30-500 mg/L of the fluorescer. Preferably, the chemiluminescent substrate solution includes 150-250 mg/L, and more preferably 200 mg/L of the fluorescer. For example, the chemiluminescent substrate solution includes 150 mg/L, 160 mg/L, 170 mg/L, 180 mg/L, 190 mg/L, 200 mg/L, 210 mg/L, 220 mg/L, 230 mg/L, 240 mg/L, and 250 mg/L of the fluorescer.

In some embodiments, the chemiluminescent substrate solution includes 1-10 g/L of the water-soluble polymeric quaternary ammonium salt. Preferably, the chemiluminescent substrate solution includes 3-7 g/L, and more preferably 5 g/L of the water-soluble polymeric quaternary ammonium salt. For example, the chemiluminescent substrate solution includes 3 g/L, 4 g/L, 5 g/L, 6 g/L, and 7 g/L of the water-soluble polymeric quaternary ammonium salt.

In a specific embodiment, the chemiluminescent substrate solution includes 50-500 mg/L, preferably 150-250 mg/L of ADP-STAR or CDP-STAR, 30-500 mg/L, preferably 150-250 mg/L of the fluorescein, and 1-10 g/L, preferably 3-7 g/L of the water-soluble polymeric quaternary ammonium salt.

In another specific embodiment, the chemiluminescent substrate solution includes 50-500 mg/L, preferably 150-250 mg/L of ADP-STAR, 30-500 mg/L, preferably 150-250 mg/L of the fluorescein or a carboxyl-substituted fluorescein, and 1-10 g/L, preferably 3-7 g/L of the water-soluble polymeric quaternary ammonium salt.

The chemiluminescent substrate solution further includes a buffer, a preservative, and other additives.

The type of the buffer is not particularly limited in the present disclosure, and all conventionally applicable buffers can be used in the chemiluminescent substrate solution of the present disclosure. In some embodiments, the buffer may be selected from a Tris buffer solution, an AMP buffer solution (AMP is 2-amino-2-methylpropanol), an AMPD buffer solution (AMPD is 2-amino-2-methyl-1,3-propanediol), a DEA buffer solution (DEA is diethanolamine), a CHES buffer solution (CHES is 2-(N-cyclohexylamino)ethanesulfonic acid), a Mopso buffer solution, an imidazole buffer solution, a phosphoric acid buffer solution, a carbonic acid buffer solution, a malic acid buffer solution, and a glycine buffer solution, but is not limited thereto. For example, the buffer may be selected from at least one of Tris-HCl, AMP-HCl, AMPD-HCl, DEA-HCl, CHES-HCl, boric acid-NaOH, and glycine-NaOH buffer systems. Preferably, the buffer solution is selected from an AMP-HCl buffer system. The AMP-HCl buffer system may have a good buffering effect within a pH range of 9.0-10.0, and research has found that it is conducive to the stability of the chemiluminescent substrate molecules.

According to some embodiments, the pH of the buffer is 7.1-10.6, preferably about 9.0 to about 10.0, especially preferably about 9.5.

The amount of the buffer is not particularly limited in the present disclosure and can be determined depending on the selected buffer system.

The type of the preservative is not particularly limited in the present disclosure, and all preservatives conventionally used in a detection reagent can be used in the present disclosure. The examples may be: sodium azide, Proclin series, potassium sorbate, sodium benzoate, BND, erythromycin, gentamicin and the like, but are not limited thereto. Sodium azide and Proclin series are preferred.

The amount of the buffer is not particularly limited in the present disclosure and can be determined depending on the type of the selected preservative.

In some embodiments, the chemiluminescent substrate solution may further include magnesium chloride. For example, 0.1-2 mM of magnesium chloride may be included.

According to a specific embodiment, the chemiluminescent substrate solution includes 50-500 mg/L, preferably 150-250 mg/L of ADP-STAR or CDP-STAR; 30-500 mg/L, preferably 150-250 mg/L of the fluorescein; 1-10 g/L, preferably 3-7 g/L of the water-soluble polymeric quaternary ammonium salt; a proper amount (e.g., 30-500 mM) of the AMP-HCl buffer solution; and a proper amount (e.g., 0.5-2 g/L) of sodium azide, with a pH of about 9.0 to about 10.0, such as about 9.5.

In another specific embodiment, the chemiluminescent substrate solution includes 50-500 mg/L, preferably 150-250 mg/L of ADP-STAR; 30-500 mg/L, preferably 150-250 mg/L of the fluorescein or a carboxyl-substituted fluorescein; 1-10 g/L, preferably 3-7 g/L of the water-soluble polymeric quaternary ammonium salt; a proper amount (e.g., 30-500 mM) of the AMP-HCl buffer solution; and a proper amount (e.g., 0.5-2 g/L) of sodium azide, with a pH of about 9.0 to about 10.0, such as about 9.5.

According to some embodiments, the chemiluminescent substrate solution of the present disclosure has a low background luminescence value, a high luminescence efficiency, and a wide linear range, which is suitable for an alkaline-phosphatase-catalyzed luminescence system, especially applicable to chemiluminescent immunoassay.

According to some specific embodiments, the chemiluminescent substrate solution provided by the present disclosure reacts with an alkaline phosphatase with a concentration range of 0.03-400 ng/mL, and the luminescence values exhibit good linearity (R² ≥ 0.99). As shown in the examples, the background value of the chemiluminescent substrate solution may be as low as 3,000 photons/second or less, making it possible to detect an alkaline phosphatase with an ultra-low concentration reaching 10⁻¹⁹ mol/L, such that the detection sensitivity is improved. Additionally, the detection values of the chemiluminescent substrate solution may be as high as 100M photons/second or more, such as 100M-200M photons/second and 120M-200M photons/second, which exhibits significantly wider linearity. The chemiluminescent substrate solution mentioned above can be used to detect samples with high analyte concentrations without requiring additional dilution.

The chemiluminescent substrate solution of the present disclosure is especially suitable for detecting human chorionic gonadotropin (HCG) in a sample to be tested.

HCG is a glycoprotein primarily secreted by placental trophoblast cells with a low level in non-pregnant populations, usually less than 3 mIU/ml. However, the concentration of HCG may be up to about 2,000,000 mIU/ml during pregnancy. The clinical use of HCG test results helps determine pregnancy. Meanwhile, the detection of HCG during pregnancy is also of great significance. As described above, the chemiluminescent substrate solution of the present disclosure has a characteristic of wide linear luminescence, making it suitable for samples with greatly varying concentrations of analyte such as HCG.

According to some embodiments, the concentration of the HCG in the sample ranges from 1 mIU/mL to 200,000 mIU/mL.

In some embodiments, the double-antibody sandwich method is used for the detection of HCG. According to a specific embodiment, in the detection, superparamagnetic particles coated with an HCG antibody are used as a capture reagent, and an alkaline-phosphatase-labeled HCG antibody is employed as a marker. In some embodiments, the HCG antibody is a monoclonal antibody.

The chemiluminescent substrate solution of the present disclosure, when combined with the double-antibody sandwich method, exhibits high sensitivity for detecting HCG and a wide linear range and does not require additional dilution of the sample, thus simplifying operations, saving detection reagents and avoiding the interference and precision reduction caused by the additional dilution.

In the present disclosure, as for the application of the chemiluminescent substrate solution to the immunoassay of HCG, the specific form and amount of the HCG antibody, the specific capture reagent, the alkaline phosphatase marker and the other components in the corresponding immunoassay reagent are not particularly limited, and any suitable reagents can be used.

A "sample" mentioned herein, unless otherwise indicated, refers to a biological sample, which may be from a mammal, preferably a blood sample from a human, and more preferably a serum sample.

In addition to HCG, the chemiluminescent substrate solution of the present disclosure is also suitable for detecting other samples with large concentration ranges of the analyte, such as the HBsAg, and TSH, not limited to HCG.

In a third aspect of the disclosure, a chemiluminescence detection method using the aforementioned chemiluminescent substrate solution is further provided. According to an embodiment, the chemiluminescence detection method includes steps of:
mixing a sample to be tested with a detection reagent including a capture reagent and an alkaline phosphatase marker that are capable of binding to an analyte to obtain an alkaline-phosphatase-labeled immune complex;
mixing the alkaline-phosphatase-labeled immune complex with the chemiluminescent substrate solution in any embodiment mentioned above to obtain a mixed solution; and
determining the light signals of the mixed solution, and obtaining the analysis result of the sample to be tested on the basis of the light signals.

In some specific embodiments, the chemiluminescent substrate solution includes ADP-STAR or CDP-STAR as a chemiluminescent substrate, a fluorescein, and a water-soluble polymeric quaternary ammonium salt.

In another specific embodiment, the chemiluminescent substrate solution includes ADP-STAR, a fluorescein or a carboxyl-substituted fluorescein as a fluorescer, and a water-soluble polymeric quaternary ammonium salt.

In some embodiments, the chemiluminescent substrate solution includes 50-500 mg/L of ADP-STAR or CDP-STAR. Preferably, the chemiluminescent substrate solution includes 150-250 mg/L, more preferably 200 mg/L of ADP-STAR or CDP-STAR.

In some embodiments, the chemiluminescent substrate solution includes 30-500 mg/L of the fluorescein. Preferably, the chemiluminescent substrate solution includes 150-250 mg/L, and more preferably 200 mg/L of the fluorescein.

In some embodiments, the chemiluminescent substrate solution includes 1-10 g/L of the water-soluble polymeric quaternary ammonium salt. Preferably, the chemiluminescent substrate solution includes 3-7 g/L, and more preferably 5 g/L of the water-soluble polymeric quaternary ammonium salt. The water-soluble polymeric quaternary ammonium salt is as described above.

Likewise, the chemiluminescent substrate solution further includes a buffer, a preservative and other additives. The buffer, preservative and other additives are as described above, and will not be repeated herein.

According to a specific embodiment, the chemiluminescent substrate solution includes 50-500 mg/L, preferably 150-250 mg/L of ADP-STAR or CDP-STAR; 30-500 mg/L, preferably 150-250 mg/L of the fluorescein; 1-10 g/L, preferably 3-7 g/L of the water-soluble polymeric quaternary ammonium salt; a proper amount (e.g., 30-500 mM) of the AMP-HCl buffer solution; and a proper amount (e.g., 0.5-2 g/L) of sodium azide, with a pH of about 9.0 to about 10.0, such as about 9.5.

In another specific embodiment, the chemiluminescent substrate solution includes 50-500 mg/L, preferably 150-250 mg/L of ADP-STAR; 30-500 mg/L, preferably 150-250 mg/L of the fluorescein or a carboxyl-substituted fluorescein; 1-10 g/L, preferably 3-7 g/L of the water-soluble polymeric quaternary ammonium salt; a proper amount (e.g., 30-500 mM) of the AMP-HCl buffer solution; and a proper amount (e.g., 0.5-2 g/L) of sodium azide, with a pH of about 9.0 to about 10.0, such as about 9.5.

In a fourth aspect of the disclosure, a chemiluminescence detection method, as another embodiment, is further provided. The chemiluminescence detection method includes steps of:
mixing a sample to be tested with a detection reagent including a capture reagent and an alkaline phosphatase marker that are capable of binding to an analyte to obtain an alkaline-phosphatase-labeled immune complex;
mixing the alkaline-phosphatase-labeled immune complex with a chemiluminescent substrate solution; and
determining the light signals of the mixed solution, and obtaining the analysis result of the sample to be tested on the basis of the light signals;
wherein the chemiluminescent substrate solution includes a chemiluminescent substrate, a fluorescer, and a surfactant. In addition, the ratio of the number of photons per second at the upper limit of detection to that at the lower limit of detection of the chemiluminescent substrate solution is 30,000 or more; alternatively, the lower limit of linear detection of the chemiluminescent substrate solution is less than or equal to 2,000 photons/second, and the upper limit of linear detection of the photometer is larger than or equal to 100M photons/second.

In the chemiluminescence detection method, a chemiluminescent substrate solution with wide linear luminescence values is used, with the ratio of the number of photons per second at the upper limit of detection to that at the lower limit of detection being 30,000 or more, which can apply to the immunoassay of a sample in which the concentrations of the analyte differ by orders of magnitude of up to 10⁵-10⁶.

In some embodiments, the ratio of the number of photons generated per second at the upper limit of linear detection of the chemiluminescent substrate solution to that at the lower limit of linear detection thereof is 50,000 or more, even up to 60,000 or more. In some embodiments, the ratio of the number of photons generated per second at the upper limit of detection of the chemiluminescent substrate solution to that at the lower limit of detection thereof is 1,000,000 or less, such as 800,000 or less, 600,000 or less, 500,000 or less, even 400,000 or less.

Alternatively, the lower limit of linear detection of the chemiluminescent substrate solution is 1,000-3,000 photons/second, preferably 1,000-2,000 photons/second; and the upper limit of linear detection of the chemiluminescent substrate solution is 100M-200M photons/second, preferably 120M-200M photons/second.

In some embodiment, determining the light signals of the mixed solution includes determining the light signals of the mixed solution using a photometer, with the lower limit of detection of the photometer being less than or equal to 2,000 photons/second, and the upper limit of detection of the photometer being larger than or equal to 100M photons/second.

Using such a photometer may better match the wide range of luminescence values of the chemiluminescent substrate solution of the present disclosure, further simplify the detection steps, and improve the efficiency and precision of the detection.

It is understood that, as mentioned above, it is also possible for the chemiluminescence detection method of the present disclosure to achieve detection with a wide linear range by using a detection apparatus including a photometer with the other detection ranges.

In some embodiments, the chemiluminescent substrate is a dioxetane compound, and preferably is selected from chlorinated dioxetane compounds with a spiroadamantane substituent. The fluorescer is selected from a fluorescein and/or a carboxyl-substituted fluorescein. The surfactant is selected from water-soluble polymeric quaternary ammonium salt surfactants.

As mentioned above, preferably, the chlorinated dioxetane compounds with a spiroadamantane substituent are ADP-STAR and CDP-STAR. The carboxyl-substituted fluorescein is 5-carboxyfluorescein, 6-carboxyfluorescein, and 5(6)-carboxyfluorescein.

Preferably, the water-soluble polymeric quaternary ammonium salt is a polyvinyl-containing quaternary ammonium salt, preferably at least one of polyvinylbenzyl-trimethylammonium chloride, polyvinylbenzyl-benzyldimethylammonium chloride, and polyvinylbenzyl-tributylammonium chloride.

The contents of the chemiluminescent substrate, the fluorescer, and the water-soluble polymeric quaternary ammonium salt in the chemiluminescent substrate solution are as described above, and will not be repeated herein.

In some embodiments, in the detection methods of the third aspect and the fourth aspect of the present disclosure, the analyte is human chorionic gonadotropin (HCG).

In some embodiments, the concentration of HCG in the sample to be tested ranges from 1 mIU/mL to 200,000 mIU/mL. According to a specific embodiment, the method is a double-antibody sandwich method. According to a specific embodiment, the capture reagent is superparamagnetic particles coated with an HCG antibody. According to a more specific embodiment, the HCG antibody is a monoclonal antibody.

The sample to be tested is as defined above.

### Examples

Various embodiments and advantages of the disclosure are illustrated below by specific examples, but the scope of the disclosure is not limited thereby.

### Example 1: Linearity performance when using different chemiluminescent substrates

Formulation of chemiluminescent substrate solution:
Chemiluminescent substrate: 200 mg
5(6)-Carboxyfluorescein: 200 mg
Polyvinylbenzyltrimethylammonium chloride: 5 g
AMP-HCl: 50 mM
Magnesium chloride: 200 mg
Sodium azide: 1 g.
Water: up to a total volume of 1 L

A series of chemiluminescent substrate solutions 1-1 to 1-4 were prepared according to the formulation above, using the chemiluminescent substrates as shown in Table 1 below.

**Table 1**

| Chemiluminescent substrate solution | Chemiluminescent substrate |
|---|---|
| 1-1 | AMPPD |
| 1-2 | CSPD |
| 1-3 | ADP-STAR |
| 1-4 | CDP-STAR |

Tests were conducted using the aforementioned chemiluminescent substrate solutions 1-1 to 1-4 on a Mindray CL-6000i full-automatic chemiluminescence immunoassay analyzer. Alkaline phosphatase (AP) solutions with different concentration gradients were mixed with the chemiluminescent substrate solutions respectively, incubated for a period of time, and then subjected to light-signal acquisition to obtain the background signal values (i.e., the concentration of the alkaline phosphatase was 0) and luminescence signal values acquired at 2 min, respectively. The concentrations of the alkaline phosphatase in the system and the detection signal values were shown in Table 2 below.

**Table 2**

| Chemiluminescent substrate solution | 1-1 | 1-2 | 1-3 | 1-4 |
|---|---|---|---|---|
| Chemiluminescent substrate | AMPPD | CSPD | ADP-STAR | CDP-STAR |
| Concentration of AP (ng/mL) | RLU (number of photons/second) | | | |
| 0 | 4,736 | 904 | 1,681 | 807 |
| 0.03 | 21,318 | 10,592 | 21,252 | 23,163 |
| 0.5 | 268,768 | 147,952 | 314, 652 | 339,886 |
| 5 | 2,490,726 | 1,536,422 | 3,020,790 | 3,358,141 |
| 50 | 22,934,439 | 17,851,702 | 23,898,506 | 25,088,083 |
| 100 | 40,045,569 | 34,585,418 | 43,174,174 | 48,425,597 |
| 200 | 65,992,786 | 60,178,500 | 86,677,025 | 86,423,603 |
| 400 | 98,451,533 | 100,426,348 | 167,526,515 | 149,275,704 |

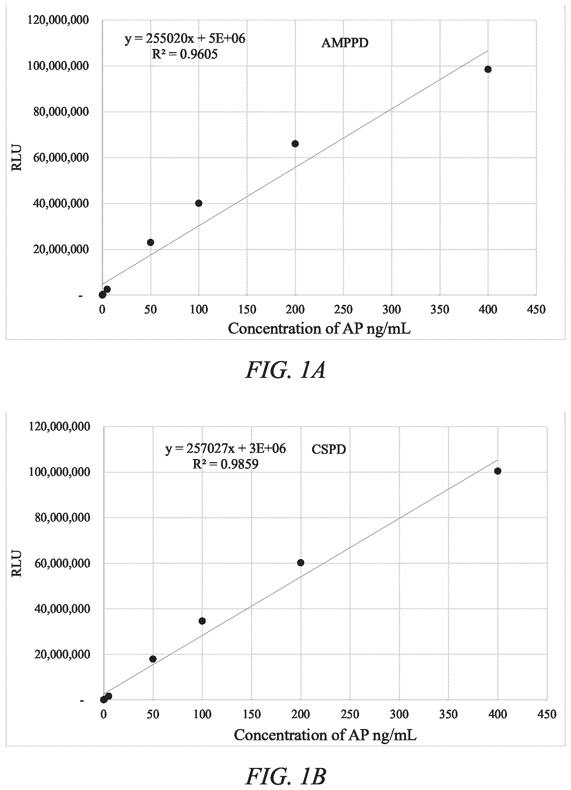

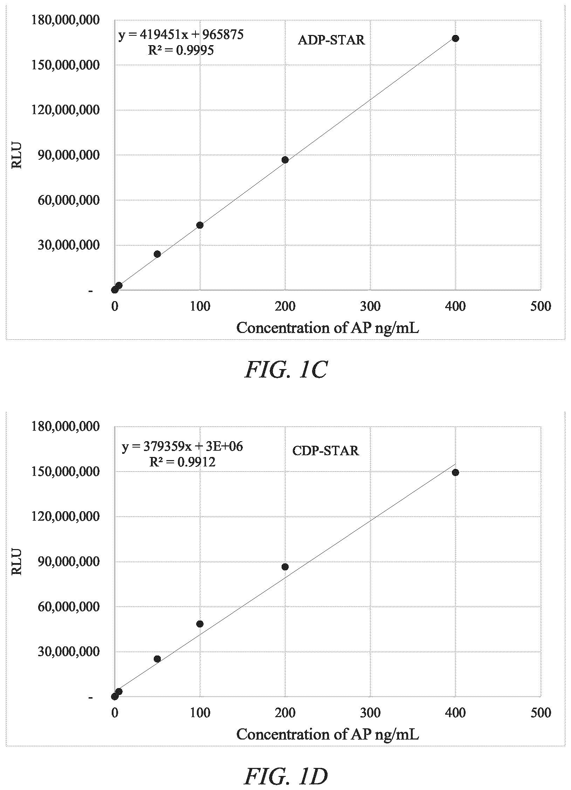

Graphs of the light signals (the number of photons/second) measured using the chemiluminescent substrate solutions 1-1 to 1-4 versus the concentration of the alkaline phosphatase in the systems were plotted and linearly fitted respectively, as shown in FIGS. 1A-1D.

As can be seen from the data in Table 2 and FIGS. 1A-1D, the chemiluminescent substrate solutions 1-2, 1-3 and 1-4 exhibited low background signals. Additionally, the chemiluminescent substrate solutions 1-3 and 1-4 also had a characteristic of high luminescence efficiency, and good linearity (R²≥0.99) within the luminescence range of the detection. Among the chemiluminescent substrates, ADP-STAR performed best.

### Example 2: Linearity performance when using ADP-STAR and different fluorescers

Formulation of chemiluminescent substrate solution:
ADP-STAR: 200 mg
Fluorescer: 200 mg
Polyvinylbenzyltrimethylammonium chloride: 5 g
AMP-HCl: 50 mM
Magnesium chloride: 200 mg
Sodium azide: 1 g.
Water: up to a total volume of 1 L

A series of chemiluminescent substrate solutions 2-1 to 2-4 were prepared according to the formulation above, using the fluorescers as shown in Table 3 below.

**Table 3**

| Chemiluminescent substrate solution | Fluorescer |
|---|---|
| 2-1 | Fluorescein |
| 2-2 | 5-Carboxyfluorescein |
| 2-3 | 6-Carboxyfluorescein |
| 2-4 | 5(6)-Carboxyfluorescein |

Tests were conducted using the aforementioned chemiluminescent substrate solutions 2-1 to 2-4 on a Mindray CL-6000i full-automatic chemiluminescence immunoassay analyzer. Alkaline phosphatase (AP) solutions with different concentration gradients were mixed with the chemiluminescent substrate solutions respectively, incubated for a period of time, and then subjected to light-signal acquisition to obtain the background signal values (i.e., the concentration of the alkaline phosphatase was 0) and luminescence signal acquired at 2 min, respectively. The concentrations of the alkaline phosphatase in the system and the detection signal values were shown in Table 4 below.

**Table 4**

| | | | | |
|---|---|---|---|---|
| Chemilumine scent substrate solution | 2-1 | 2-2 | 2-3 | 2-4 |
| Fluorescer | Fluorescein | 5-Carboxyfluore scein | 6-Carboxyfluore scein | 5(6)-Carboxyfluor escein |

| Concentratio n of AP (ng/mL) | RLU (number of photons/second) | | | |
|---|---|---|---|---|
| 0 | 1,989 | 1,405 | 1,677 | 1,681 |
| 0.03 | 17,183 | 23,458 | 24,495 | 21,252 |
| 0.5 | 262,208 | 351,701 | 361,440 | 314, 652 |
| 5 | 2,793,415 | 3,432,637 | 3,480,053 | 3,020,790 |
| 50 | 20,799,241 | 26,656,954 | 27,652,849 | 23,898,506 |
| 100 | 38,943,689 | 49,160, 767 | 49,797,710 | 43,174,174 |
| 200 | 75,942,665 | 100,407,855 | 99,525,846 | 86,677,025 |
| 400 | 131,181,845 | 184,916,513 | 190,047,810 | 167,526,515 |

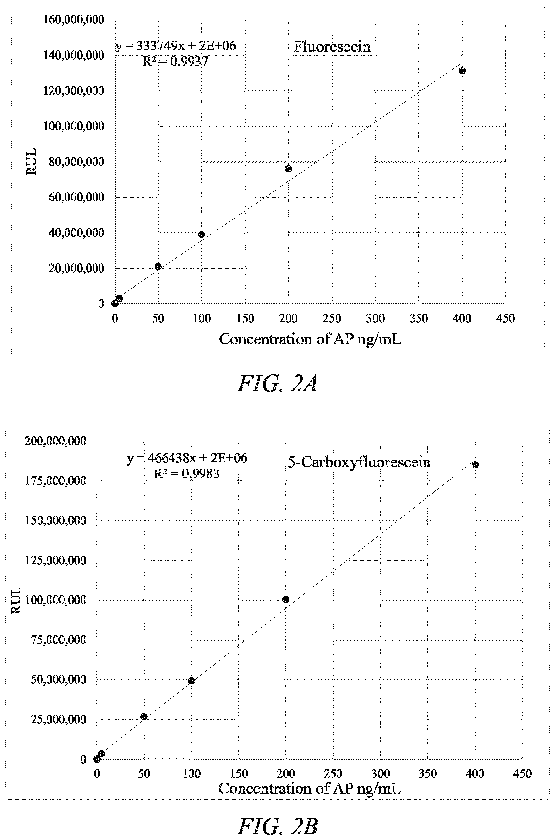

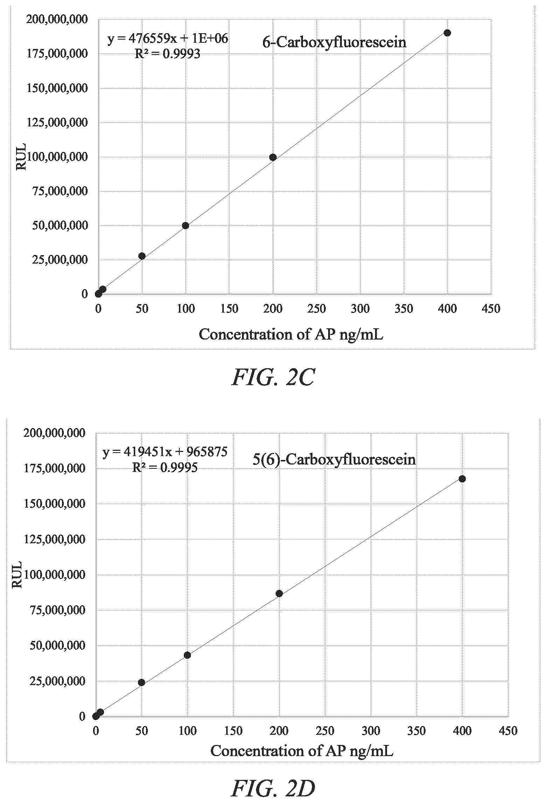

Graphs of the light signals (the number of photons/second) measured using the chemiluminescent substrate solutions 2-1 to 2-4 versus the concentration of the alkaline phosphatase in the systems were plotted and linearly fitted respectively, as shown in FIGS. 2A-2D.

As can be seen from the data in Table 4 and FIGS. 2A-2D, the chemiluminescent substrate solutions 2-1 to 2-4 all exhibited low background signals and high luminescence efficiency, and had good linearity (R²≥0.99) within the luminescence range of the detection.

### Example 3: Linearity performance when using CDP-STAR and different fluorescers

The chemiluminescent substrate solutions were prepared according to the following formulation:
CDP-STAR: 200 mg
Fluorescer: 200 mg
Polyvinylbenzyltrimethylammonium chloride: 5 g
AMP-HCl: 50 mM
Magnesium chloride: 200 mg
Sodium azide: 1 g.
Water: up to a total volume of 1 L

A series of chemiluminescent substrate solutions 3-1 to 3-4 were prepared according to the formulation above, using the fluorescers as shown in Table 5 below.

**Table 5**

| Chemiluminescent substrate solution | Fluorescer |
|---|---|
| 3-1 | Fluorescein |
| 3-2 | 5-Carboxyfluorescein |
| 3-3 | 6-Carboxyfluorescein |
| 3-4 | 5(6)-Carboxyfluorescein |

Tests were conducted using the aforementioned chemiluminescent substrate solutions 3-1 to 3-4 on a Mindray CL-6000i full-automatic chemiluminescence immunoassay analyzer. Alkaline phosphatase (AP) solutions with different concentration gradients were mixed with the chemiluminescent substrate solutions respectively, incubated for a period of time, and then subjected to light-signal acquisition to obtain the background signal values (i.e., the concentration of the alkaline phosphatase was 0) and luminescence signal values acquired at 2 min, respectively. The concentrations of the alkaline phosphatase in the system and the detection signal values were shown in Table 6 below.

**Table 6**

| Chemiluminescent substrate solution | 3-1 | 3-2 | 3-3 | 3-4 |
|---|---|---|---|---|
| Fluorescer | Fluorescein | 5-Carboxyflu orescein | 6-Carboxyfluor escein | 5(6)-Carboxyfluo rescein |
| Concentration of AP (ng/mL) | RLU (number of photons/second) | | | |
| 0 | 1,389 | 798 | 1,013 | 807 |
| 0.03 | 18,728 | 25,567 | 26,964 | 23,163 |
| 0.5 | 283,236 | 379,907 | 390,426 | 339,886 |
| 5 | 3,105,373 | 3,815,982 | 3,868,692 | 3,358,141 |
| 50 | 21,834,549 | 27,983,836 | 29,029,303 | 25,088,083 |
| 100 | 43,680,543 | 54,140,360 | 55,854,776 | 48,425,597 |
| 200 | 75,720,627 | 97,292,611 | 101, 103, 837 | 86,423,603 |
| 400 | 131,890,525 | 176,771,186 | 179,343,466 | 149,275,704 |

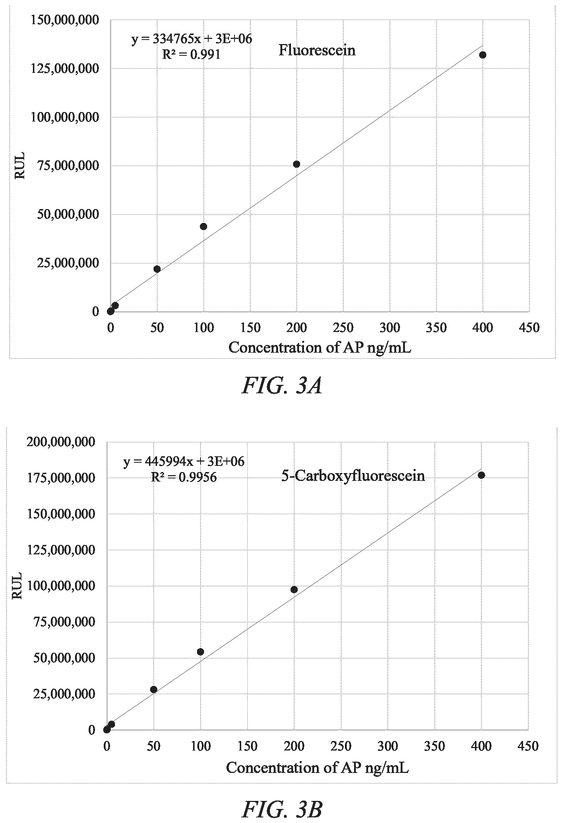

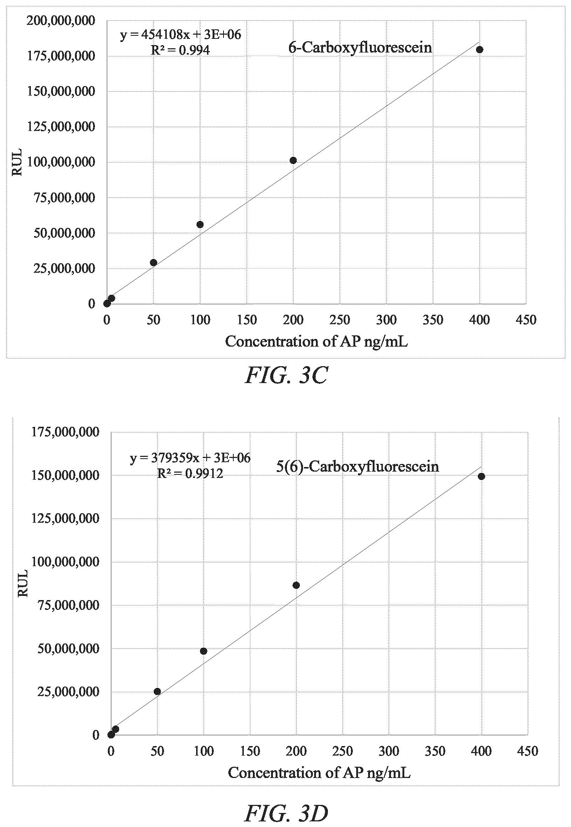

Graphs of the light signals (the number of photons/second) measured using the chemiluminescent substrate solutions 3-1 to 3-4 versus the concentration of the alkaline phosphatase in the systems were plotted and linearly fitted respectively, as shown in FIGS. 3A-3D.

As can be seen from the data in Table 6 and FIGS. 3A-3D, the chemiluminescent substrate solutions 3-1 to 3-4 all exhibited low background signals and high luminescence efficiency, and had good linearity (R²≥0.99) within the luminescence range of the detection.

### Example 4: Linearity performance when using ADP-STAR and different cationic surfactants of quaternary ammonium salts

Formulation of chemiluminescent substrate solution:
ADP-STAR: 200 mg
Fluorescein: 200 mg
Quaternary ammonium salt surfactant: 5 g
AMP-HCl: 50 mM
Magnesium chloride: 200 mg
Sodium azide: 1 g.
Water: up to a total volume of 1 L

A series of chemiluminescent substrate solutions 4-1 to 4-4 were prepared according to the formulation above, using the cationic surfactants of quaternary ammonium salts as shown in Table 7 below.

**Table 7**

| Chemiluminescent substrate solution | Cationic surfactant of quaternary ammonium salt |
|---|---|
| 4-1 | Polyvinylbenzyltrimethylammonium chloride (TMQ) |
| 4-2 | Polyvinylbenzyltributylammonium chloride (TBQ) |
| 4-3 | Polyvinylbenzyl(benzyldimethyl)ammonium chloride (BDMQ) |
| 4-4 | Cetylammonium bromide (CTAB) |

Tests were conducted using the aforementioned chemiluminescent substrate solutions 4-1 to 4-4 on a Mindray CL-6000i full-automatic chemiluminescence immunoassay analyzer. Alkaline phosphatase (AP) solutions with different concentration gradients were mixed with the chemiluminescent substrate solutions respectively, incubated for a period of time, and then subjected to light-signal acquisition to obtain the background signal values (i.e., the concentration of the alkaline phosphatase was 0) and luminescence signal values acquired at 2 min, respectively. The concentrations of the alkaline phosphatase in the system and the detection signal values were shown in Table 8 below.

**Table 8**

| Chemiluminescent substrate solution | 4-1 | 4-2 | 4-3 | 4-4 |
|---|---|---|---|---|
| Surfactant | TMQ | TBQ | BDMQ | CTAB |
| Concentration of AP ng/mL | RLU | | | |
| 0 | 1,989 | 1,855 | 1,907 | 5,896 |
| 0.03 | 17,183 | 24,636 | 20,380 | 7,380 |
| 0.5 | 262,208 | 303,083 | 276,118 | 27,858 |
| 5 | 2,793,415 | 2,826,997 | 2,559,818 | 224, 219 |
| 50 | 20,799,241 | 22,560,126 | 23,037,041 | 2,176,864 |
| 100 | 38,943,689 | 39,291,832 | 37,954,693 | 4,660,162 |
| 200 | 75,942,665 | 81,990,581 | 66,690,051 | 9,380,900 |
| 400 | 131,181,845 | 150,218,696 | 136,498,234 | 13,793,862 |

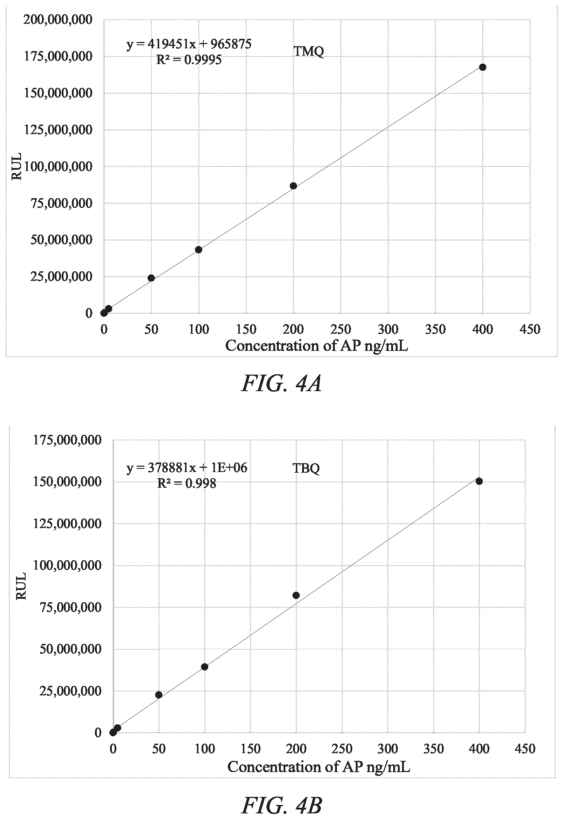

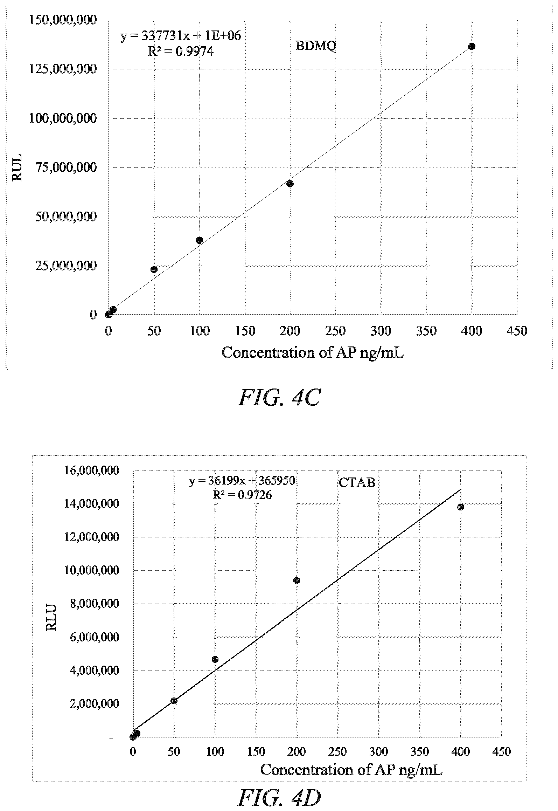

Graphs of the light signals (the number of photons/second) measured using the chemiluminescent substrate solutions 4-1 to 4-4 versus the concentration of the alkaline phosphatase in the systems were plotted and linearly fitted respectively, as shown in FIGS. 4A-4D.

As can be seen from the data in Table 8 and FIGS. 4A-4D, the chemiluminescent substrate solutions 4-1 to 4-3 had low background signals and high luminescence efficiency, and had good linearity (R²≥0.99) within the luminescence range of the detection. The chemiluminescent substrate solution 4-4 had high background signals and low luminescence efficiency, and cannot maintain good linearity (R²<0.99) within the luminescence range of the detection. Additionally, the ratio of the light signals at the upper limit of detection to those at the lower limit of detection was small (less than 3,000).

### Example 5: Linearity performance when using CDP-STAR and different cationic surfactants of quaternary ammonium salts

Formulation of chemiluminescent substrate solution:
CDP-STAR: 200 mg
Fluorescein: 200 mg
Quaternary ammonium salt surfactant: 5 g
AMP-HCl: 50 mM
Magnesium chloride: 200 mg
Sodium azide: 1 g.
Water: up to a total volume of 1 L

A series of chemiluminescent substrate solutions 5-1 to 5-4 were prepared according to the formulation above, using the cationic surfactants of quaternary ammonium salts as shown in Table 9 below.

**Table 9**

| Chemiluminescent substrate solution | Cationic surfactant of quaternary ammonium salt |
|---|---|
| 5-1 | Polyvinylbenzyltrimethylammonium chloride (TMQ) |
| 5-2 | Polyvinylbenzyltributylammonium chloride (TBQ) |
| 5-3 | Polyvinylbenzyl(benzyldimethyl)ammonium chloride (BDMQ) |
| 5-4 | Cetylammonium bromide (CTAB) |

Tests were conducted using the aforementioned chemiluminescent substrate solutions 5-1 to 5-4 on a Mindray CL-6000i full-automatic chemiluminescence immunoassay analyzer. Alkaline phosphatase (AP) solutions with different concentration gradients were mixed with the chemiluminescent substrate solutions respectively, incubated for a period of time, and then subjected to light-signal acquisition to obtain the background signal values (i.e., the concentration of the alkaline phosphatase was 0) and luminescence signal values acquired at 2 min, respectively. The concentrations of the alkaline phosphatase in the system and the detection signal values were shown in Table 10 below.

**Table 10**

| Chemiluminescent substrate solution | 5-1 | 5-2 | 5-3 | 5-4 |
|---|---|---|---|---|
| Surfactant | TMQ | TBQ | BDMQ | CTAB |
| Concentration of AP (ng/mL) | RLU | | | |
| 0 | 1,389 | 1,259 | 1,347 | 4,840 |
| 0.03 | 18,728 | 21,879 | 22,814 | 7,395 |
| 0.5 | 283,236 | 288,667 | 324, 416 | 28,192 |
| 5 | 3,105,373 | 2,548,347 | 2,807,865 | 227,582 |
| 50 | 21,834,549 | 22,423,689 | 23,834,003 | 2,187,748 |
| 100 | 43,680,543 | 39,859,769 | 39,541,610 | 4,697,443 |
| 200 | 75,720,627 | 70,385,061 | 67,415,324 | 8,562,138 |
| 400 | 131,890,525 | 129,302,663 | 123,413,073 | 12,642,130 |

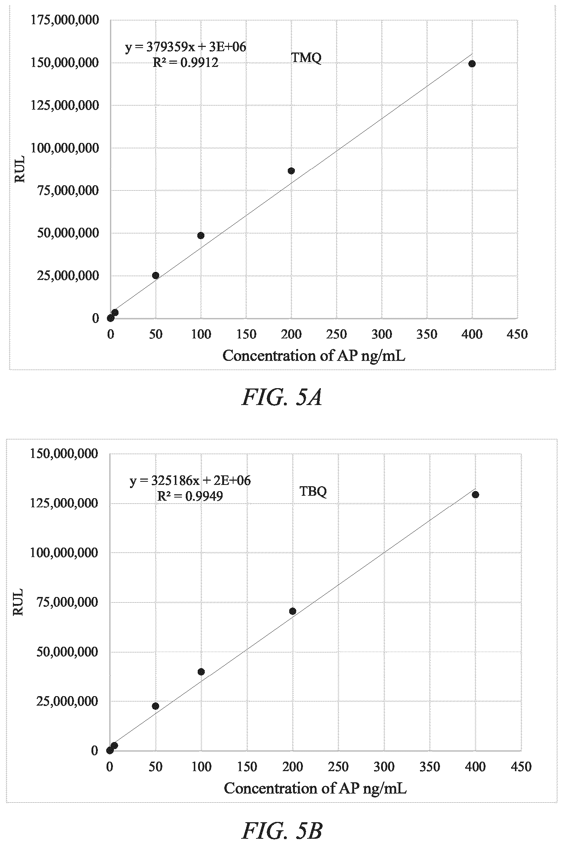

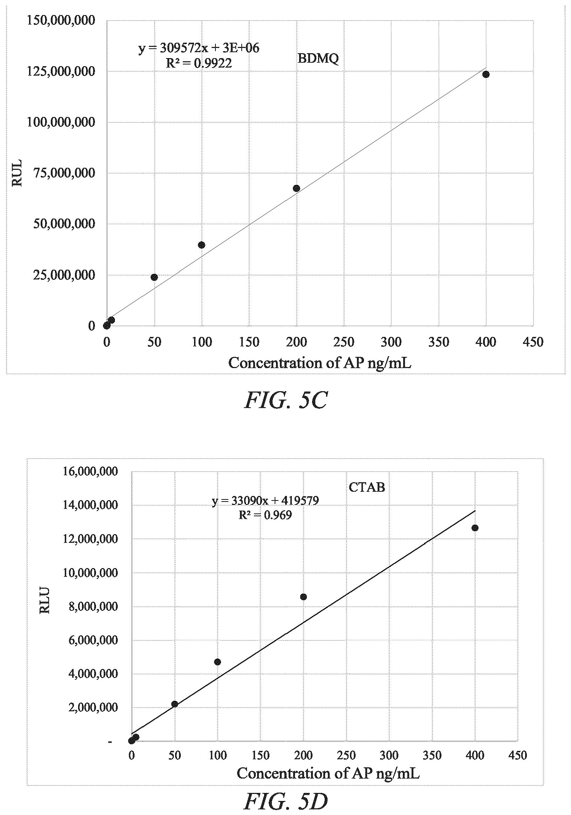

Graphs of the light signals (the number of photons/second) measured using the chemiluminescent substrate solutions 5-1 to 5-4 versus the concentration of the alkaline phosphatase in the systems were plotted and linearly fitted respectively, as shown in FIGS. 5A-5D.

As can be seen from the data in Table 10 and FIGS. 5A-5D, the chemiluminescent substrate solutions 5-1 to 5-3 had low background signals and high luminescence efficiency and had good linearity (R²≥0.99) within the luminescence range of detection. The chemiluminescent substrate solution 5-4 had high background signals and low luminescence efficiency and cannot maintain good linearity (R²<0.99) within the luminescence range of detection. Additionally, the ratio of the light signals at the upper limit of detection to those at the lower limit of detection was small (less than 3,000).

### Example 6: Wide linear detection of HCG

The chemiluminescent substrate solution 6-1 was prepared according to the following formulation:
ADP-STAR: 200 mg
Fluorescein: 200 mg
Polyvinylbenzyltrimethylammonium chloride: 5 g
AMP-HCl: 50 mM
Magnesium chloride: 200 mg
Sodium azide: 1 g
Water: up to a total volume of 1 L

The chemiluminescent substrate solution 6-2 was prepared according to the following formulation:
CDP-STAR: 200 mg
Fluorescein: 200 mg
Polyvinylbenzyltrimethylammonium chloride: 5 g
AMP-HCl: 50 mM
Magnesium chloride: 200 mg
Sodium azide: 1 g
Water: up to a total volume of 1 L

The prepared serum samples with different HCG concentrations were tested using the aforementioned chemiluminescent substrate solutions 6-1 to 6-2 on a Mindray CL-6000i full-automatic chemiluminescence immunoassay analyzer. The concentration of the samples ranged from 1 mIU/mL to 200,000 mIU/mL, and the HCG detection kit was the Total β Human Chorionic Gonadotropin (Total β HCG) detection kit from Mindray (chemiluminescence immunoassay). The samples were incubated with the chemiluminescent substrate solutions 6-1 and 6-2, respectively, and after incubating for 2 minutes, the light signals (the number of photons/second) were acquired and recorded, and the concentrations of HCG in the samples were calculated according to the light signals and denoted as back-calculated concentration. The relative deviations of the back-calculated concentrations were calculated relative to the nominal concentrations of HCG in the samples, and the results were shown in Tables 11 and 12 below.

**Table 11**

| Chemiluminescent substrate solution 6-1 | | | | |
|---|---|---|---|---|
| Sample no. | Concentration (mIU/mL) | RLU (number of photons/second) | Back-calculated concentration (mIU/mL) | Back-calculation deviation (%) |
| 1 | 1.07 | 3,169 | 1.12 | 4.5% |
| 2 | 1.74 | 3,808 | 1.78 | 2.7% |
| 3 | 6.87 | 8,670 | 6.84 | 0.4% |
| 4 | 33.15 | 33,366 | 32.53 | -1.9% |
| 5 | 132.14 | 128,877 | 131.95 | -0.1% |
| 6 | 637.19 | 603,127 | 625.85 | -1.8% |
| 7 | 1316.63 | 1,256,165 | 1306.37 | -0.8% |
| 8 | 3110.32 | 3,083,802 | 3212.81 | 3.3% |
| 9 | 7134.88 | 6,099,186 | 6892.68 | 3.4% |
| 10 | 25346.79 | 22,892,719 | 26507.43 | 4.6% |
| 11 | 121368.48 | 85,727,382 | 117081.93 | 3.5% |
| 12 | 177347.37 | 110,385,488 | 169155.73 | 4.6% |
| 13 | 203006.72 | 130,563,615 | 205715.52 | 1.3% |

**Table 12**

| Chemiluminescent substrate solution 6-2 | | | | |
|---|---|---|---|---|
| Sample no. | Concentration (mIU/mL) | RLU (number of photons/second) | Back-calculated concentration (mIU/mL) | Back-calculation deviation (%) |
| 1 | 1.42 | 2,888 | 1.43 | 0.3% |
| 2 | 2.32 | 3,752 | 2.28 | -1.5% |
| 3 | 7.66 | 9,361 | 7.82 | 2.1% |
| 4 | 47.27 | 50,004 | 47.86 | 1.3% |
| 5 | 217.41 | 217,823 | 212.80 | -2.1% |
| 6 | 1046.56 | 1,041,432 | 1020.40 | -2.5% |
| 7 | 4790.76 | 5,033,768 | 4696.25 | 2.0% |
| 8 | 5495.31 | 5,917,926 | 5634.95 | 2.5% |
| 9 | 23070.90 | 19,067,766 | 23389.04 | 1.4% |
| 10 | 35387.87 | 25,765,982 | 34396.67 | -2.8% |
| 11 | 56101.46 | 37,826,834 | 56620.89 | 0.9% |
| 12 | 212909.72 | 103, 479, 543 | 213549.24 | 0.3% |

As can be seen from Tables 11 and 12, for the detection of the HCG item, when the concentration of HCG in samples ranged from 1 mIU/mL to 200,000 mIU/mL, the chemiluminescent substrate solutions of the present disclosure can be directly used for detection and analysis without dilution, and the deviations of the detection results relative to nominal concentration are all within ±5%.

The description above merely relates to the examples of some of the embodiments of the disclosure, and is not intended to limit the scope of the disclosure. All equivalent structure transformations made by using the contents of the specification and the drawings of the disclosure from the concept of the disclosure, or the direct/indirect applications of the contents in other related technical fields are all included in the patent protection scope of the disclosure.

## Claims

1. A chemiluminescent substrate solution, **characterized in that** the chemiluminescent substrate solution comprises a chemiluminescent substrate, a fluorescein, and a water-soluble polymeric quaternary ammonium salt, wherein the chemiluminescent substrate is selected from chlorinated dioxetane compounds with a spiroadamantane substituent.

2. The chemiluminescent substrate solution of claim 1, **characterized in that** the chlorinated dioxetane compounds with a spiroadamantane substituent are ADP-STAR and CDP-STAR.

3. The chemiluminescent substrate solution of claim 1, **characterized in that** the water-soluble polymeric quaternary ammonium salt is selected from polyvinyl-containing quaternary ammonium salts, preferably from at least one of polyvinylbenzyl-trimethylammonium chloride, polyvinylbenzyl-benzyldimethylammonium chloride, and polyvinylbenzyl-tributylammonium chloride.

4. The chemiluminescent substrate solution of any one of claims 1-3, **characterized in that** a ratio of a number of photons generated per second at an upper limit of linear detection of the chemiluminescent substrate solution to that at a lower limit of linear detection thereof is 30,000 or more, preferably 50,000 or more.

5. The chemiluminescent substrate solution of any one of claims 1-3, **characterized in that** the lower limit of linear detection of the chemiluminescent substrate solution is less than or equal to 3,000 photons/second, and the upper limit of linear detection of the chemiluminescent substrate solution is larger than or equal to 100M photons/second.

6. The chemiluminescent substrate solution of claim 5, **characterized in that** the lower limit of linear detection of the chemiluminescent substrate solution is 1,000-3,000 photons/second, preferably 1,000-2,000 photons/second; and the upper limit of linear detection of the chemiluminescent substrate solution is 100M-200M photons/second, preferably 120M-200M photons/second.

7. The chemiluminescent substrate solution of any one of claims 1-6, **characterized in that** the chemiluminescent substrate solution comprises 50-500 mg/L of the chemiluminescent substrate, 30-500 mg/L of the fluorescein, and 1-10 g/L of the water-soluble polymeric quaternary ammonium salt.

8. The chemiluminescent substrate solution of claim 7, **characterized in that** the chemiluminescent substrate solution comprises 150-250 mg/L of the chemiluminescent substrate, 150-250 mg/L of the fluorescein, and 3-7 g/L of the water-soluble polymeric quaternary ammonium salt.

9. The chemiluminescent substrate solution of any one of claims 1-8, **characterized in that** the chemiluminescent substrate solution is chemiluminescent through the catalysis of an alkaline phosphatase, and preferably a concentration of the alkaline phosphatase is as low as 10⁻¹⁹ mol/L.

10. The chemiluminescent substrate solution of any one of claims 1-9, **characterized in that** the chemiluminescent substrate solution is used to detect human chorionic gonadotropin (HCG) in a sample to be tested and preferably the concentration of the human chorionic gonadotropin (HCG) in the sample to be tested ranges from 1 mIU/mL to 200,000 mIU/mL, and preferably the sample to be tested is a blood sample.

11. A chemiluminescence detection method, **characterized in that** the chemiluminescence detection method comprises:
mixing a sample to be tested with a detection reagent comprising a capture reagent and an alkaline phosphatase marker that are capable of binding to an analyte to obtain an alkaline-phosphatase-labeled immune complex;
mixing the alkaline-phosphatase-labeled immune complex with a chemiluminescent substrate solution to obtain a mixed solution; and
determining light signals of the mixed solution, and obtaining an analysis result of the sample to be tested on the basis of the light signals;
wherein the chemiluminescent substrate solution comprises a chemiluminescent substrate, a fluorescer, and a surfactant; and
wherein a ratio of a number of photons generated per second at an upper limit of linear detection of the chemiluminescent substrate solution to that at a lower limit of linear detection thereof is 30,000 or more, or
a lower limit of linear detection of the chemiluminescent substrate solution is less than or equal to 2,000 photons/second, and an upper limit of linear detection of the chemiluminescent substrate solution is larger than or equal to 100M photons/second.

12. The method of claim 11, **characterized in that** the ratio of the number of photons generated per second at the upper limit of linear detection of the chemiluminescent substrate solution to that at the lower limit of linear detection thereof is 50,000 or more, or
the lower limit of linear detection of the chemiluminescent substrate solution is 1,000-3,000 photons/second, preferably 1,000-2,000 photons/second, and the upper limit of linear detection of the chemiluminescent substrate solution is 100M-200M photons/second, preferably 120M-200M photons/second.

13. The method of claim 11 or 12, **characterized in that** determining the light signals of the mixed solution comprises determining the light signals of the mixed solution using a photometer, with a lower limit of linear detection of the photometer being less than or equal to 2,000 photons/second, and an upper limit of linear detection of the photometer being larger than or equal to 100M photons/second.

14. The method of any one of claims 11-13, **characterized in that** the chemiluminescent substrate is a dioxetane compound, and preferably is selected from chlorinated dioxetane compounds with a spiroadamantane substituent; the fluorescer is selected from a fluorescein and/or a carboxyl-substituted fluorescein; and the surfactant is selected from water-soluble polymeric quaternary ammonium salt surfactants; and the chlorinated dioxetane compounds with a spiroadamantane substituent are preferably ADP-STAR and CDP-STAR; and the carboxyl-substituted fluorescein is preferably 5-carboxyfluorescein, 6-carboxyfluorescein, and 5(6)-carboxyfluorescein, and the water-soluble polymeric quaternary ammonium salt surfactants is preferably polyvinylbenzyl-trimethylammonium chloride, polyvinylbenzyl-benzyldimethylammonium chloride, or polyvinylbenzyl-tributylammonium chloride.

15. The method of any one of claims 1-14, **characterized in that** the analyte is human chorionic gonadotropin (HCG); preferably, the concentration of the HCG in the sample to be tested ranges from 1 mIU/mL to 200,000 mIU/mL; more preferably, the method is a double-antibody sandwich method; and more preferably, the capture reagent is superparamagnetic particles coated with an HCG antibody.
